# EUROPÄISCHE PATENTSCHRIFT

(11) **EP 3 721 923 B1**
(45) Veröffentlichungstag und Bekanntmachung des Hinweises auf die Patenterteilung: **04.11.2026**
(21) Anmeldenummer: 20168767.0
(22) Anmeldetag: 08.04.2020
(51) Int. Cl.: A61M 5/315, A61M 5/48, A61M 5/00, A61M 5/24, A61M 5/31

(54) **AUSPRESSVORRICHTUNG FÜR SPRITZEN**
EJECTION DEVICE FOR SYRINGES
DISPOSITIF D'EXTRACTION POUR PULVÉRISATEURS

(30) Priorität: 10.04.2019 DE 102019205162
(43) Veröffentlichungstag der Anmeldung: 14.10.2020
(73) Patentinhaber: Heiden, Dr. Klemens, 51588 Nürnbrecht (DE)
(72) Erfinder: Heiden, Dr. Klemens, 51588 Nürnbrecht (DE)
(74) Vertreter: dompatent

(56) Entgegenhaltungen:
- EP-A1- 2 783 753
- WO-A2-2016/138018
- US-A1- 2004 035 743

## Beschreibung

Die Erfindung betrifft eine Auspressvorrichtung für Spritzen sowie die Verwendung einer Ausvorrichtung zum Auspressen von Spritzen, insbesondere zur Durchführung einer Sterilfiltration eines in einer Spritze befindlichen Fluides oder einer halbfesten Zubereitung.

Bei der Herstellung von Arzneimitteln nach einer bestimmten Rezeptur sind eine Reihe von Anforderungen, wie beispielsweise die Partikelfreiheit und die Freiheit von Mikroorganismen des hergestellten Arzneimittels zu erfüllen. Dabei ist die Sterilfiltration ein Standardverfahren, das insbesondere in öffentlichen Apotheken, Krankenhausapotheken und auch in Laboratorien angewendet wird.

Ausgangspunkt bei einer Sterilfiltration ist in der Regel eine Spritze, in der die gewünschten Bestandteile eines Arzneimittels aufgenommen sind. Um das in der Spritze befindliche Arzneimittel der Sterilfiltration zu unterziehen, kommen im Allgemeinen Spritzenvorsatzfilter zum Einsatz, die - im Falle der weit verbreiteten Luer-Lock-Spritzen - auf die Öffnung der Spitze geschraubt werden. Der Filter in einem Spritzenvorsatzfilter ist in der Regel durch eine Membran mit einem hinreichend geringen Porendurchmesser gebildet, deren Material abhängig von den Eigenschaften des Arzneimittels (hydrophil oder lipophil) variiert. Wird das Arzneimittel durch den Spritzenvorsatzfilter gedrückt, werden Mikroorganismen, insbesondere Bakterien, aus dem Arzneimittel herausgefiltert.

Damit das Arzneimittel den Spritzenvorsatzfilter passiert, muss insbesondere bei höher viskosen Fluiden der hohe Widerstand des Spritzenvorsatzfilters überwunden werden. Um einen hinreichen hohen Druck in der Spritze zu erzeugen, ist der Spritzenkolben mit einer großen Kraft zu drücken. Insbesondere ist die erforderliche Kraft während der gesamten Dauer der Sterilfiltration, also bis das Arzneimittel den Spitzenvorsatzfilter vollständig durchlaufen hat, aufzubringen. Die Sterilfiltration wird in der Regel durch einen Apotheker oder einen pharmazeutisch-technischen Assistenten durchgeführt und beansprucht einen nicht unerheblichen Kraft- und Zeitaufwand. Die Sterilfiltration von 5 ml eines Arzneimittels kann erfahrungsgemäß rund 20 Minuten benötigen, abhängig von der Viskosität des Arzneimittels und von den physischen Fähigkeiten der Person, die die Sterilfiltration durchführt. Nach der Beendigung der Sterilfiltration ist nach dem Deutschen Arzneimittel-Kodex und dem Europäischen Arzneibuch die ordnungsgemäße Funktionsweise und Integrität des Filters mithilfe des Bubble-Point-Tests zu prüfen. Dazu wird der Spritzenvorsatzfilter nach erfolgter Sterilfiltration von der ausgepressten Spritze abgeschraubt, anschließend wird eine vorbestimmte Menge Luft (z.B. 10ml) in die leere Spritze eingesaugt. Anschließend wird der Spritzenvorsatzfilter erneut aufgeschraubt und mit einer Spritzenkanüle versehen. Das Ende dieser Spritzenkanüle wird in ein mit Wasser gefülltes Becherglas unter die Wasseroberfläche gehalten. Zur Durchführung des Bubble-Point-Tests wird durch Drücken auf den Spritzenkolben die Luft in der Spritze komprimiert und gegen die mit dem vorherigen Filtrat gefüllten Poren der Filtermembrane gedrückt. In Abhängigkeit der Oberflächenspannung des zuvor filtrierten Fluides und dem Porendurchmesser des Spritzenvorsatzfilters ergeben sich beim Drücken zwischen 1,4 und 7 bar, bis das Fluid von der komprimierten Luft aus den Filterporen ausgepresst wird. Dieser Moment kann durch einen kontinuierlichen Austritt von Luftblasen aus der Kanülenspitze unter der Wasseroberfläche im Becherglas erkannt werden. Durch das Ausblasen der Luft sinkt der Innendruck in der Spritze bzw. der zu überwindende Widerstand in dem Spritzenvorsatzfilter, da die Luft durch die freien Filterporen entweichen kann. Je nach Arzneimittel darf erst ab einer Kompression um einen vorgegebenen Faktor, beispielsweise um den Faktor 5-6, der Austritt eines kontinuierlichen Luftblasenstroms erkennbar sein. Bei einem nicht bestandenen Bubble-Point-Test ist die Sterilfiltration mit einem neuen Spritzenvorsatzfilter erneut durchzuführen. Wie bei der Sterilfiltration, muss auch zur Durchführung des Bubble-Point-Tests eine sehr hohe Kraft (beispielsweise 2,5 - 6,5 bar, 60 - 140 N) auf den Spritzenkolben ausgeübt werden, wobei gleichzeitig darauf geachtet werden muss, bei welchem Kompressionsfaktor ein Luftblasenstrom erkennbar ist. Das Ergebnis des Bubble-Point-Tests ist ferner im Herstellungsprotokoll des Arzneimittels zu vermerken.

Zuvor wird die mittels des Bubble-Point-Tests festgestellte korrekte Funktionsweise und Integrität des Spritzenvorsatzfilters durch denjenigen bestätigt, der den Bubble-Point-Test durchführt und im Herstellungsprotokoll vermerkt, jedoch kann ein durchgeführter Bubble-Point-Test nachträglich nicht noch einmal reproduziert werden, so dass die ordnungsgemäße Durchführung des Bubble-Point-Tests vertrauensbasiert und im Streitfall kaum nachweisbar ist und in keiner Weise dem heutigen Dokumentationsstandard entspricht. Die genaue Erfassung des Bubble-Point Drucks kann bisher nur absolut unzulänglich erfasst und nicht richtig dokumentiert werden.

Es besteht somit ein Bedarf, die vorgenannten Probleme zu lösen, insbesondere die Durchführung einer Sterilfiltration und eines Bubble-Point-Tests zu erleichtern.

Eine Auspressvorrichtung für Spritzen mit den Merkmalen des Oberbegriffs des Anspruch 1, die jedoch für eine schrittweise Betätigung einer Spritze zum Ausgeben mehrerer Dosen einer medizinischen Flüssigkeit vorgesehen ist, ist aus US 2004/035743 A1 bekannt. Eine ähnliche Auspressvorrichtung ist in WO 2016/138018 A2 offenbart.

Die erfindungsgemäße Auspressvorrichtung für Spritzen ist definiert durch die Merkmale des Anspruchs 1.

Die erfindungsgemäße Auspressvorrichtung für Spritzen mit einem Spritzenzylinder und einem Spritzenkolben weist eine Halterung zum Haltern einer Spritze auf, wobei die Halterung des Spritzenzylinders der Spritze zumindest teilweise umgreift. Ferner weist die Auspressvorrichtung ein Spritzendrückelement auf, das am freien Ende des Spritzenkolbens angreift, wobei der Spritzenkolben zum Auspressen der Spritze durch das Spritzendrückelement drückbar ist. Zur Aufbringung einer kontinuierlichen auf das Spritzendrückelement und somit auf den Spritzenkolben wirkenden Auspresskraft zum Auspressen der Spritze ist eine Vorschubvorrichtung vorgesehen, die das Spritzendrückelement vorschiebt, wobei eine Betätigungsvorrichtung die Vorschubvorrichtung betätigt. Mit anderen Worten, die von der Betätigungsvorrichtung betätigte Vorschubvorrichtung bewirkt eine Vorschubbewegung des Spritzendrückelements zur Erzeugung der Auspresskraft.

Die Vorschubbewegung kann kontinuierlich über einen vorgegebenen Zeitraum oder eine vorgegebene Wegstrecke erfolgen, in dem die Vorschubvorrichtung von der Betätigungsvorrichtung kontinuierlich bewegt wird. Die Vorschubbewegung kann auch als einmalige definierte Bewegung erfolgen, wobei ein Kraftspeicher betätigt wird, der anschließend über einen längeren Zeitraum das Spritzendrückelement vorschiebt. Mit anderen Worten, die Vorschubvorrichtung weist einen Kraftspeicher auf. Der Kraftspeicher wird bei Betätigung der Vorschubvorrichtung beladen und anschließend erzeugt der Kraftspeicher die Vorschubbewegung des Spritzendrückelements zur Erzeugung der Auspresskraft kontinuierlich über einen längeren Zeitraum, beispielsweise bis zum vollständigen Ausdrücken der Spritze. Die Spritze kann formschlüssig in der Halterung aufgenommen sein. Die Halterung bildet ein Gegenlager für die Auspresskraft während des Auspressvorgangs bzw. während der Vorschubbewegung des Spritzenkolbens. Die Halterung kann ferner einen Anschlag für das Spritzendrückelement bilden, um die Vorschubbewegung des Spritzendrückelements zu stoppen.

Die Auspressvorrichtung kann ferner eine Halterungsvorrichtung, beispielsweise einen Ständer mit einem oder mehreren Halterungselementen aufweisen, an der die Halterung, die Vorschubvorrichtung und die Betätigungsvorrichtung befestigt sind. Vorzugsweise ist die Spritze dabei vertikal ausgerichtet und die Auspressvorrichtung wirkt in vertikaler Richtung.

Die vorgesehene Anordnung ermöglicht es einem Benutzer der Vorrichtung, eine Spritze auszudrücken, ohne den Spritzenkolben der Spritze manuell über die gesamte Dauer des Auspressvorgangs drücken zu müssen. Der Benutzer der Auspressvorrichtung kann die Betätigungsvorrichtung zu Beginn des gewünschten Auspressvorgangs betätigen, wobei kontinuierlich eine Vorschubbewegung zur Erzeugung der Auspresskraft zum Auspressen der Spritze erzeugt wird. Erfindungsgemäß kann der Benutzer mittels der Betätigungsvorrichtung eine in der Vorschubvorrichtung gespeicherte Kraft erzeugen, die anschließend kontinuierlich zur Erzeugung der Auspresskraft zum Auspressen der Spritze freigegeben wird.

Gemäß der Erfindung weist die Vorschubvorrichtung ein Vorschubelement und eine Kraftspeichereinheit mit einem Kraftspeicherelement als Kraftspeicher auf, wobei das Vorschubelement die Kraftspeichereinheit betätigt, um eine Kraft in das Kraftspeicherelement einzuleiten.

Der Benutzer der Auspressvorrichtung kann somit die Zeit über die Dauer des Auspressvorgangs anderweitig nutzen. Es ist nicht erforderlich kontinuierlich manuell auf den Spritzenkolben zu drücken, sondern es genügt eine Betätigung der Betätigungsvorrichtung zu Beginn eines Auspressvorgangs.

Ferner kann die Betätigungsvorrichtung einen Kraftverstärker aufweisen, beispielsweise in Form eines Hebels oder Getriebes. Bei der Betätigung der Betätigungsvorrichtung ist somit im Vergleich zu der manuellen direkten Betätigung der Spritze, d.h. ohne die erfindungsgemäße Auspressvorrichtung, ein geringerer Kraftaufwand notwendig.

Je nach Ausgestaltung der Betätigungsvorrichtung kann im Vergleich manuellen direkten Betätigung der Spritze, eine wesentlich höhere Auspresskraft erzeugt werden, so dass auf einfache Art und Weise die Sterilfiltration auch an höher viskosen Fluiden - beispielsweise Öle, Salben oder Gele - durchgeführt werden kann.

Vorzugsweise weist die Halterung eine erste Auflagefläche auf, an der die Spritze mit einem vorderen Ende des Spritzenzylinders oder mit einem Kragen am hinteren Ende des Spritzenzylinders aufliegen kann. Dadurch wird in vorteilhafter Weise ein Gegenlager für die Auspresskraft gebildet.

Beispielsweise kann die Halterung eine Durchgangsbohrung zur Aufnahme der Spritze aufweisen. Die erste Auflagefläche kann beispielsweise in der Durchgangsbohrung durch eine Durchmesserverjüngung der Durchgangsbohrung gebildet sein. Durch die Durchgangsbohrung ist die Spritze in vorteilhafterweise halterbar, wobei die Durchgangsbohrung die Spritze seitlich umgreift und somit stabil in Position hält.

Vorzugsweise kann die durch die Durchgangsbohrung gebildete Innenwandung die Außenwandung des Spritzenzylinders stabilisieren, wodurch verhindert werden kann, dass sich die Außenwandung des Spritzenzylinders bei höheren Filtrationsdrücken nach außen wölbt, wodurch die Gefahr entstehen würde, das das auszupressende Fluid durch einen durch Wölbung der Außenwandung und des Spritzenzylinders entstehenden Spalt zwischen Kolben und Spritzenzylinder entweichen kann. Dabei ist vorzugsweise vorgesehen, dass die Durchgangsbohrung einen an den Außendurchmesser des Spritzenzylinders angepassten Durchmessers aufweist.

Die Halterung kann einen ersten Anschlag aufweisen, wobei zwischen dem ersten Anschlag und der ersten Auflagefläche in einer ersten Richtung ein erster Abstand gebildet ist. Die erste Richtung der Halterung ist dabei im befestigten Zustand der Halterung die Richtung der Vorschubbewegung des Spritzendrückelements.

Der an der Halterung vorgesehene erste Anschlag definiert den Endpunkt eines Auspressvorgangs, in dem die Bewegung des Spritzendrückelements durch den ersten Anschlag begrenzt ist. Das Spritzendrückelement kann den Spritzenkolben nur soweit in die Spritze drücken, bis der erste Anschlag erreicht ist. Der Abstand zwischen der ersten Auflagefläche und dem ersten Anschlag kann eine in der Spritze verbleibende Restmenge des Fluides bestimmen. Mit anderen Worten: bei einer in der Halterung aufgenommenen auszupressende Spritze wird der Spritzenzylinder durch die erste Auflagefläche der Halterung axial abgestützt. Der erste Anschlag ist mit dem ersten Abstand von der erste Auflagefläche beabstandet, sodass beispielsweise der Spritzenkolben nicht vollständig in die Spritze gedrückt werden kann und die Spritze nur teilweise ausgepresst wird. Durch die Wahl eines geeigneten ersten Abstandes zwischen dem ersten Anschlag und der ersten Auflagefläche kann somit festgelegt werden, welche Menge eines in der Spritze befindlichen Fluides ausgepresst wird. Selbstverständlich kann der erste Abstand auch so gewählt sein, dass die Spritze vollkommen ausgedrückt wird. Dabei kann vorgesehen sein, dass das Spritzendrückelement nicht an den ersten Anschlag anschlägt, sondern die Bewegung durch das vollständige Einschieben des Spritzenkolbens in den Spritzenzylinder begrenzt wird, wobei dann bei vollständig eingeschobener Spritze ein Spalt zwischen Spritzendrückelement und erstem Anschlag verbleiben kann.

Ferner kann vorgesehen sein, dass die Halterung eine zweite Auflagefläche aufweist, an der die Spritze mit einem vorderen Ende des Spritzenzylinders oder mit einem Kragen am hinteren Ende des Spritzenzylinders aufliegen kann. Die Halterung kann dabei zu Nutzung der zweiten Auflagefläche verdreht werden.

Die zweite Auflagefläche kann in der Durchgangsbohrung durch eine Durchmesserverjüngung der Durchgangsbohrung gebildet sein, wobei die erste und zweite Auflagefläche in entgegengesetzte Richtung gerichtet sind.

Alternativ kann die Halterung auch eine zweite Durchgangsbohrung zur Aufnahme der Spritze aufweisen. Die zweite Auflagefläche kann dann beispielsweise in der zweiten Durchgangsbohrung durch eine Durchmesserverjüngung der zweiten Durchgangsbohrung gebildet sein.

Die Halterung kann einen zweiten Anschlag aufweisen, wobei zwischen dem zweiten Anschlag und der zweiten Auflagefläche in einer zweiten Richtung ein zweiter Abstand gebildet ist. Die zweite Richtung der Halterung ist dabei im befestigten Zustand der Halterung die Richtung der Vorschubbewegung des Spritzendrückelements. Bei einem Ausführungsbeispiel, bei dem die erste und die zweite Auflagefläche in einer Durchgangsbohrung angeordnet sind, entspricht die erste Richtung der zweiten Richtung.

Bei einem Ausführungsbeispiel, bei dem die erste und die zweite Auflagefläche in einer Durchgangsbohrung angeordnet sind, sind der erste und der zweite Anschlag an unterschiedlichen Enden der Durchgangsbohrung angeordnet.

Vorzugseise ist der zweite Abstand größer als der erste Abstand ist.

Beispielsweise kann der erste Abstand so gewählt sein, dass die Spritze beim Anliegen an der ersten Auflagefläche von dem Spritzendrückelement vollständig ausdrückbar ist, und der zweite Abstand so gewählt sein, dass die Spritze beim Anliegen an der zweiten Auflagefläche von dem Spritzendrückelement nicht vollständig ausdrückbar ist.

Bei einem Ausführungsbeispiel, bei dem die erste und die zweite Auflagefläche in einer Durchgangsbohrung angeordnet sind, kann die Halterung bei Bedarf umgedreht werden. Dies ermöglicht es in vorteilhafter Weise zwischen zwei verschiedenen Endpunkten eines Auspressvorgangs zu wählen und, je nach Bedarf, eine andere Menge eines in der Spritze befindlichen Fluides auszupressen.

Optional kann vorgesehen sein, dass zwischen der Halterung und dem Spritzendrückelement eine oder mehrere Distanzscheiben mit einer jeweils definierten Dicke anordenbar sind, wobei die oder mehrere Distanzscheiben vorzugsweise eine Aussparung aufweisen. Durch die Anordnung einer oder mehrerer Distanzscheiben ist die auszupressende Menge eines in der Spritze befindlichen Fluides variierbar. Die eine oder mehrere Distanzscheiben können beispielsweise auf dem ersten oder zweiten Anschlag aufgelegt werden. Dabei kann bzw. können die Durchgangsbohrung bzw. die zweite Durchgangsbohrung und die Aussparung bzw. die Aussparungen miteinander fluchten. Die äußerste Distanzscheibe bildet einen neuen Anschlag, so dass durch die Distanzscheiben der Abstand zwischen der entsprechenden ersten oder zweiten Auflagefläche und dem Anschlag veränderbar ist. Die eine oder mehrere Distanzscheiben können auch an dem Spritzendrückelement angeordnet sein.

Vorzugsweise haben die Distanzscheiben eine Dicke von 5 oder 10 Millimetern. Beispielsweise kann die Auspressvorrichtung insgesamt drei Distanzscheiben umfassen, wobei eine der Distanzscheiben eine Dicke von 10 Millimetern ausweist und zwei der Distanzscheiben eine Dicke von 5 Millimetern aufweisen. Die Distanzscheiben können einzeln oder miteinander kombiniert verwendet werden.

Durch das Vorsehen von Distanzscheiben kann die Position des Anschlags verändert werden. Dadurch kann präzise festgelegt werden, wie weit der Spritzenkolben vom Spritzendrückelement vorgeschoben werden kann. Bei Spritzen, die mit einer vorgegebenen Menge Fluid befüllt sind, kann somit festgelegt werden, welche Menge des Fluides ausgepresst wird, ohne dass eine dauernde Aufsicht während des Auspressvorgangs erforderlich ist.

Die eine oder mehreren Distanzscheiben sind vorzugsweise an der Halterung angeordnet. Durch das Anordnen der Distanzscheiben an der Halterung, insbesondere an dem Anschlag der Halterung, kann die Anpassung des Arbeitsbereichs zum Drücken des Spritzenkolbens auf einfache Art und Weise erfolgen.

Es kann vorgesehen sein, dass das Vorschubelement durch eine Vorschubstange gebildet ist. Die Betätigungsvorrichtung kann formschlüssig und/oder kraftschlüssig mit dem Vorschubelement zusammengreifen, um die Vorschubbewegung zu erzeugen.

Die Kraftspeichereinheit kann ferner ein Führungselement aufweisen. Das Kraftspeicherelement ist vorzugsweise eine Druckfeder.

Durch diese Anordnung wird auf einfache Weise ermöglicht, dass die für die Funktionsweise der Auspressvorrichtung erforderliche Kraft gespeichert und zum Auspressen der Spritze kontinuierlich freigegeben werden kann.

Ferner kann vorgesehen sein, dass das Spritzendrückelement eine erste Vertiefung aufweist, in die das Kraftspeicherelement zumindest teilweise aufgenommen ist.

Durch die erste Vertiefung, in der das Kraftspeicherelement aufgenommen, wird das Kraftspeicherelement geführt. Das Kraftspeicherelement wirkt beispielsweise zwischen einer Fläche des Führungselements und einer sich in der ersten Vertiefung befindlichen Fläche des Spritzendrückelements, kann nur soweit zusammengedrückt werden, bis das Spritzendrückelement an das Führungselement anstößt. Zu diesem Zeitpunkt ist das Kraftspeicherelement vollständig in der ersten Vertiefung aufgenommen. Mit anderen Worten: Das Kraftspeicherelement kann nur in einem vorbestimmten Maße zusammengedrückt werden. Dadurch ist die in dem Kraftspeicherelement maximal speicherbare potenzielle Energie, die anschließend zur Erzeugung der Auspresskraft ausgebbar ist, stets gleich, sodass ein Auspressvorgang in vorteilhafter Weise reproduzierbar ist.

Vorzugsweise ist vorgesehen, dass das Führungselement einen Aufnahmeraum aufweist, in dem das Spritzendrückelement teilweise aufgenommen und geführt ist. Vorzugsweise weist das Führungselement ein sich in axialer Richtung erstreckendes seitlich angeordnetes Langloch auf, das in den Aufnahmeraum mündet. Das Spritzendrückelement weist vorzugsweise eine seitliche Bohrung auf, wobei das Spritzendrückelement durch ein in der seitlichen Bohrung angeordnetes Führungsteil im Langloch geführt ist.

Die Anordnung bietet den Vorteil, dass sich das Spritzendrückelement ausschließlich in einem vorgesehenen definierten Arbeitsbereich relativ zum Führungselement bewegen kann. Dadurch wird ein unkontrolliertes Herausfallen des Spritzendrückelements aus dem Führungselement vermieden.

Ferner kann vorgesehen sein, dass das Spritzendrückelement eine zweite Vertiefung aufweist, in der der Spritzenkolben teilweise aufgenommen ist.

Durch die teilweise Aufnahme des Spritzenkolbens wird der Spritzenkolben gezielt in die gewünschte Richtung gedrückt. Ferner wird vermieden, dass das Spritzendrückelement in eine Schräglage gedrückt wird.

In einem Ausführungsbeispiel der vorliegenden Erfindung weist die Auspressvorrichtung eine Sensorvorrichtung zur Bestimmung der Auspresskraft auf.

Die Sensorvorrichtung ist vorzugsweise an der Halterungsvorrichtung, an einem der Halterungselementen der Halterungsvorrichtung, an der Halterung oder an dem Spritzendrückelement angeordnet.

Durch das Vorsehen einer Sensorvorrichtung können Daten über die Auspresskraft während eines Auspressvorgangs ermittelt werden, die anschließend verarbeitet und/oder gespeichert werden. Insbesondere können die Daten der Sensorvorrichtung zu einer grafischen Darstellung verarbeitet werden. Ferner können die von der Sensorvorrichtung ermittelten Daten für eine Warnfunktion verwendet werden, um den Benutzer der Auspressvorrichtung zu warnen, wenn sich die von der Sensorvorrichtung aufgenommenen Messwerte nicht in einem definierten Bereich befinden.

Durch das Vorsehen einer Sensorvorrichtung und der Messung der Auspresskraft kann insbesondere die Dokumentation des Bubble-Point-Tests in vorteilhafter Weise verbessert werden. Insbesondere können die aufgenommenen Messwerte gespeichert und im Herstellungsprotokoll eines Arzneimittels zum Beispiel tabellarisch und auch grafisch vermerkt werden. Durch diese Prozessdokumentation kann nachträglich die ordnungsgemäße Durchführung des Bubble-Point-Tests und das Ergebnis des Tests nachgewiesen werden.

Beispielsweise kann die Sensorvorrichtung an einem der Halterungselemente der Halterungsvorrichtung angeordnet sein, wobei die Halterung auf die Sensorvorrichtung drückt. Häufig werden die Messwerte nur für die Auswertung des Bubble-Point-Tests verwendet. Um zu verhindern, dass die Sensorvorrichtung während eines Auspressdurchgangs dauerhaft belastet ist, kann die "normale" Halterung beispielsweise im Gebrauch nicht mit der Sensorvorrichtung zusammenwirken und eine spezielle Halterung wird für den Bubble-Point-Test verwendet, wobei die spezielle Halterung mit der Sensorvorrichtung zusammenwirkt. Beispielsweise kann die spezielle Halterung einen Vorsprung aufweisen, der auf die Sensorvorrichtung drückt. Alternativ kann vorgesehen sein, dass die spezielle Halterung mittels eines Halteelements an der Halterungsvorrichtung angebracht ist und mit der Sensorvorrichtung zusammenwirkt und die Halterung mit einem anderen Halteelement an der Halterungsvorrichtung angebracht ist und nicht mit der Sensorvorrichtung zusammenwirkt. Das Halteelement für die spezielle Halterung kann auch durch die Sensorvorrichtung gebildet sein. Beispielsweise kann die Sensorvorrichtung durch eine Messgabel gebildet werden, in die die spezielle Halterung eingehängt wird. Die spezielle Halterung kann einen ähnlichen Aufbau wie die Halterung aufweisen und mit einer Durchgangsbohrung für die Spritze und mit einer Auflagefläche versehen sein.

Gemäß einer weiteren bevorzugten Ausführungsform der vorliegenden Erfindung weist das Spritzendrückelement eine Markierung zur Überwachung eines Auspressvorgangs und/oder zur Anzeige eines Anfangszustandes für einen Auspressvorgang auf. Die Markierung hebt einen bestimmten Abschnitt oder Bereich des Spritzendrückelements optisch hervor und kann beispielsweise durch eine Umlaufnut gebildet sein.

Durch das Vorsehen einer Markierung am Spritzendrückelement wird dem Benutzer die visuelle Überwachung des Fortschritts des Auspressvorgangs erleichtert, indem die relative Position des Spritzendrückelements in Bezug zu den übrigen Bauteilen der Auspressvorrichtung auf einfache Art und Weise ermittelt werden kann.

Ferner kann vorgesehen sein, dass das Führungselement eine oder mehrere seitliche in den Aufnahmeraum mündende Bohrungen aufweist, die mit der Markierung des Spritzendrückelements zusammenwirken, wobei die relative Position der Markierung bzw. des Spritzendrückelements in Bezug auf das Führungselement durch die seitlichen Bohrungen sichtbar ist.

Durch das Zusammenwirken der am Spritzendrückelement vorgesehenen Markierung und den seitlichen Bohrungen des Führungselements kann der Benutzer der Auspressvorrichtung den Fortschritt des Auspressvorgangs bzw. den Anfangs- und Endzustand des Auspressvorgangs ablesen. Beispielsweise kann der Anfangszustand zum Auspressen einer definierten Menge eines Fluides aus der Spritze, bei dem das Kraftspeicherelement ausreichend beladen ist, dadurch erkannt werden, dass die Markierung vollständig durch die seitlichen Bohrungen des Führungselements sichtbar ist.

Insbesondere können die optischen Markierungen als Anzeige eines maximalen Auspressdrucks für eine eingelegte Spritze dienen. Bei Verwendung der Spritze mit einem Spritzenvorsatzfilter kann somit ein maximaler Filtrationspressdruck eingestellt werden, so dass weder die Spritze, noch das Gehäuse des angeschraubten Spritzenvorsatzfilters über die zulässige Druckbelastungsgrenze beansprucht werden. Es kann somit erkannt werden, wenn das Kraftspeicherelement mit einer ausreichenden bzw. maximal zulässigen Kraft beladen ist. Dadurch kann die Gefahr des Berstens von Spritzenzylinder, Spritzenkolben oder des vorgeschraubten Spritzenvorsatzfilters verringert werden.

Gemäß einer weiteren Ausführungsform der vorliegenden Erfindung weist die Betätigungsvorrichtung eine elektrische Antriebseinheit auf, die die Vorschubvorrichtung betätigt.

Das Vorsehen der elektrischen Antriebseinheit vereinfacht die Handhabung der Auspressvorrichtung dahingehend, dass der Benutzer die für den Auspressvorgang erforderliche Kraft nicht manuell mittels der Betätigungsvorrichtung aufbringen muss. Die Antriebseinheit kann vielmehr von dem Benutzer gesteuert werden und eine vom Benutzer festgelegte Kraft in die Vorschubvorrichtung einbringen.

Es kann ferner vorgesehen sein, dass die Vorschubvorrichtung eine Gewindespindel aufweist, die von der elektrischen Antriebseinheit angetrieben wird und das Spritzendrückelement vorschiebt.

Durch das Vorsehen einer Gewindespindel, die durch die elektrische Antriebseinheit angetrieben wird, ist in vorteilhafter Weise ein präzises Betätigen des Spritzendrückelements möglich. Insbesondere kann die wirkende Auspresskraft präzise festgelegt werden.

Zusätzlich zum Vorsehen einer elektrischen Antriebseinheit, die die Vorschubvorrichtung betätigt, kann die Auspressvorrichtung die Sensorvorrichtung zur Messung der Auspresskraft aufweisen.

Grundsätzlich kann die Sensorvorrichtung in vorteilhafter Weise, beispielsweise mit einer Signalverarbeitungseinheit, einer Anzeigevorrichtung oder einer Warnvorrichtung zusammenwirken. Insbesondere kann über die durch die Sensorvorrichtung ermittelten Messwerte eine präzise Steuerung des Auspressvorgangs sowie eine Dokumentation der wirkenden Kräfte erfolgen.

Die Halterungsvorrichtung kann eine Ständerung und einen Träger aufweisen wobei an dem Träger die Halterung, die Vorschubvorrichtung mit Spitzendrückelement und die Betätigungsvorrichtung vorzugsweise über Halteelemente befestigt sind. Der Träger kann verschiebbar an der Ständerung gelagert sein und in einer Position arretieren. Somit ist eine Höhenverstellung möglich.

Vorzugsweise wird die erfindungsgemäße Auspressvorrichtung zur Durchführung einer Sterilfiltration eines in einer Spritze befindlichen Fluides verwendet, wobei die Spritze einen Spritzenvorsatzfilter aufweist.

Ferner wird die erfindungsgemäße Auspressvorrichtung vorzugsweise zur Durchführung eines Bubble-Point-Tests verwendet.

Im Folgenden wird unter Bezugnahme auf die nachfolgenden Figuren die Erfindung anhand einer bevorzugten Ausführungsform näher erläutert.

Es zeigen:
- Fig. 1: eine perspektivische Darstellung der erfindungsgemäßen Auspressvorrichtung einer bevorzugten Ausführungsform der vorliegenden Erfindung,
- Fig. 2: eine schematische Seitenansicht der erfindungsgemäßen Auspressvorrichtung gemäß der bevorzugten Ausführungsform der vorliegenden Erfindung,
- Fig. 3: eine schematische Schnittdarstellung des Spritzendrückelements gemäß der bevorzugten Ausführungsform der vorliegenden Erfindung,
- Fig. 4: eine schematische Schnittdarstellung der Halterung gemäß der bevorzugten Ausführungsform der vorliegenden Erfindung,
- Fig. 5: eine schematische Schnittdarstellung des Führungselements gemäß der bevorzugten Ausführungsform der vorliegenden Erfindung,
- Fig. 6a: eine schematische Darstellung einer ersten möglichen Ausrichtung der Halterung und einer möglichen Anordnung von Distanzscheiben nach der bevorzugten Ausführungsform der vorliegenden Erfindung, und
- Fig. 6b: eine schematische Darstellung einer zweiten möglichen Ausrichtung der Halterung und einer möglichen Anordnung von Distanzscheiben nach der bevorzugten Ausführungsform der vorliegenden Erfindung.

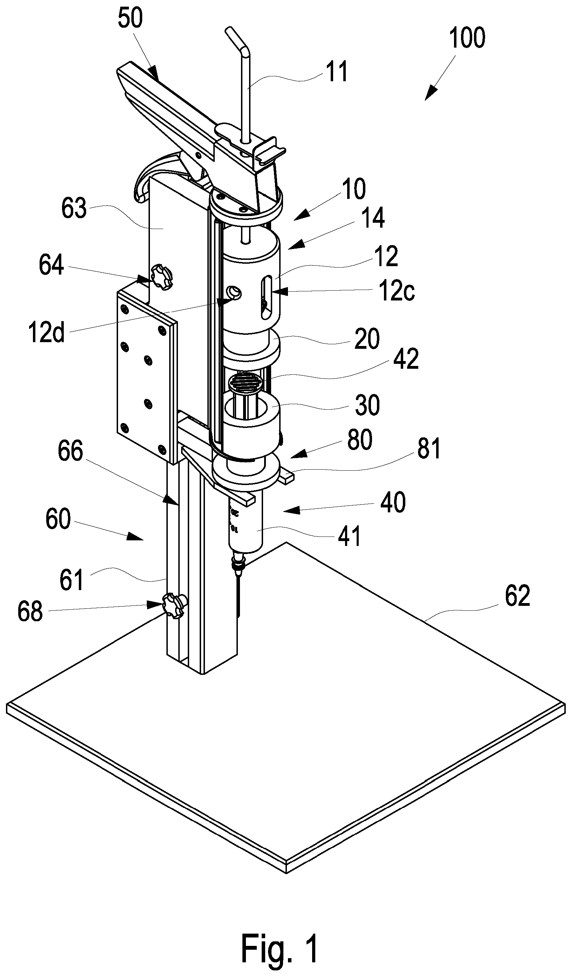

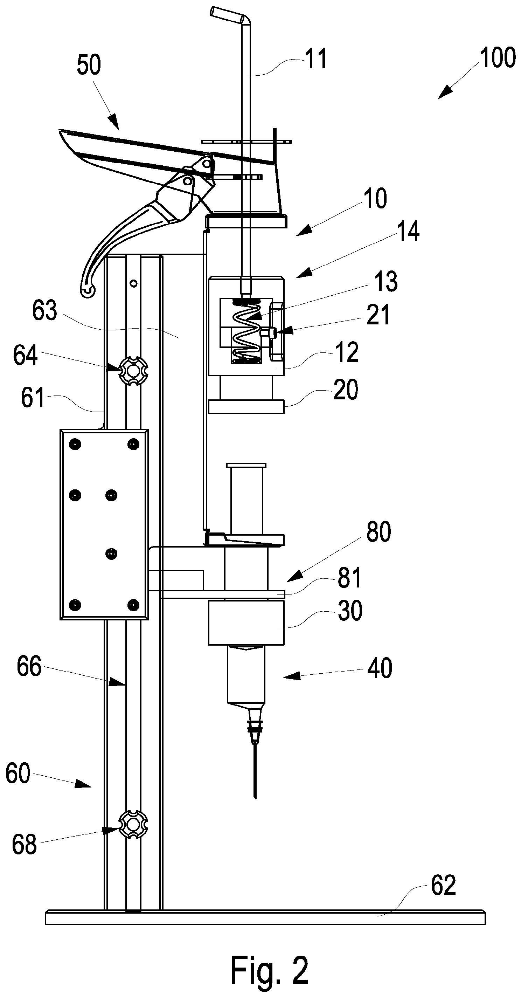

In Fig. 1 ist die bevorzugte Ausführungsform der erfindungsgemäßen Auspressvorrichtung 100 perspektivisch dargestellt. Figur 2 zeigt eine entsprechende schematische Seitenansicht der Auspressvorrichtung 100, wobei zur Verdeutlichung einige verdeckte Teile gezeigt sind.

Die Auspressvorrichtung 100 weist eine Halterung 30 auf, in die eine Spritze 40 einhängbar ist. In der Halterung 30 ist die Spritze 40 mit einem Spritzenzylinder 41 und einem Spritzenkolben 42 formschlüssig aufgenommen. Ferner weist die Auspressvorrichtung 100 ein Spritzendrückelement 20 auf, das in axialer Richtung oberhalb der Halterung 30 angeordnet ist. Oberhalb des Spritzendrückelements 20 ist ferner die Vorschubvorrichtung 10 mit einer Kraftspeichereinheit 14 und einem Vorschubelement 11 angeordnet. Die Kraftspeichereinheit 14 umfasst ein Führungselement 12 und ein Kraftspeicherelement 13, wobei das Spritzendrückelement 20 in dem Führungselement 12 in einem Aufnahmeraum 12a teilweise aufgenommen und geführt ist. Der axiale Bewegungsspielraum des Spritzendrückelements 20 im Führungselement 12 ist durch eine Gewindeschraube 21 begrenzt, die in das Spritzendrückelement 20 geschraubt und in einem Langloch 12c des Führungselements 12 geführt ist. Das Kraftspeicherelement 13 ist zwischen dem Spritzendrückelement 20 und dem Führungselement 12 angeordnet und als eine Druckfeder ausgebildet. Das Kraftspeicherelement 13 ist in einer ersten Vertiefung 20a aufgenommen. Das Vorschubelement 11 ist an dem Führungselement 12 befestigt und durch eine Vorschubstange gebildet.

An dem Vorschubelement 11 ist eine Betätigungsvorrichtung 50 vorgesehen, die kraftschlüssig und/oder formschlüssig mit dem Vorschubelement 11 zusammengreift, um einen Vorschub der Vorschubvorrichtung 10 zu erzeugen. Die Betätigungsvorrichtung 50 kann dabei eine Hebelmechanik aufweisen, über die eine Kraftverstärkung erfolgt. Durch den Vorschub, der durch eine Betätigung der Betätigungsvorrichtung 50 erzeugbar ist, wird das Kraftspeicherelement 13 zwischen einer Fläche des Führungselements 12 und einer Fläche des Spritzendrückelements 20 zusammengedrückt. Während eines Auspressvorgangs wird eine Auspresskraft kontinuierlich auf den Spritzenkolben 42 der Spritze 40 aufgebracht, indem das Kraftspeicherelement 13 entladen wird und das Spritzendrückelements 20 kontinuierlich vorschiebt. Dadurch wird ein in der Spritze 40 befindliches Fluid ausgepresst.

Bei einer Verwendung mit einem Spritzenvorsatzfilter und anschließender Filterintegritätsprüfung mittels eines Bubble-Point-Tests wird durch diesen Vorgang mittels des Kraftspeicherelements 13 die Kraft für die Komprimierung der Luft in der Spritze zur Erzeugung eines Überdrucks erzeugt.

Die Halterung 30, die Vorschubvorrichtung 10 und die Betätigungsvorrichtung 50 sind an einer Halterungsvorrichtung 60 angeordnet.

Die Halterungsvorrichtung 60 weist eine Ständerung 61 mit Standfuß 62 und einen Träger 63 auf. An dem Träger 63 sind die Halterung 30, die Vorschubvorrichtung 10 mit Spitzendrückelement 20 und die Betätigungsvorrichtung 50 über Halteelemente befestigt. Der Träger 63 ist verschiebbar an der Ständerung 61 gelagert und lässt sich durch Lösen einer Klemmschraube 64 vertikal verschieben sowie mittels der Klemmschraube 64 in einer Position arretieren. Die Lagerung kann beispielsweise durch eine Nut 66 in der Ständerung 61 erfolgen, in der der Träger 63 geführt ist und in die die Klemmschraube 64 eingreift. Somit ist eine Höhenverstellung möglich.

In der Nut 66 kann eine verstellbare Anschlageinrichtung 68 angeordnet sein. Die Anschlageinrichtung 68 bildet einen Anschlag bei der Verstellung des Trägers 63 gegenüber der Ständerung. 61 Der über die Anschlageinrichtung 68 definierbare Anschlagpunkt erlaubt eine reproduzierbare Absenkung des Trägers 63 in vertikaler Richtung und somit der Spritze, so dass eine Arbeitshöhe, die für ein Auspressen geeignet oder vorgegeben ist, reproduzierbar einstellbar ist. Die Arbeitshöhe kann beispielsweise eine Höhe sein, bei der ein Auslass einer Spritze oder einer daran direkt oder indirekt befestigten Kanüle in vorteilhafter Weise in ein Gefäß positionierbar ist. Bei der Durchführung des Bubble-Point-Tests kann der Anschlag so eingestellt sein, dass die Kanülenspitze beim Absenken des Trägers 63 unter der Wasseroberfläche im Becherglas gelangt. Die Anschlageinrichtung 68 kann beispielsweise eine Schraube aufweisen, mit der die Anschlageinrichtung 68 in der Nut 66 klemmbar ist.

Die Auspressvorrichtung 100 weist ferner eine an der Halterungsvorrichtung 60 angeordnete Sensorvorrichtung 80 auf, die die auf den Spritzenkolben 42 wirkende Auspresskraft während eines Auspressvorgangs bestimmt. Die Sensorvorrichtung 80 kann während einer Filtration mittels an der Spritze 40 angeordneten Spritzenvorsatzfilter oder bei einem Filterintegritätstest verwendet werden.

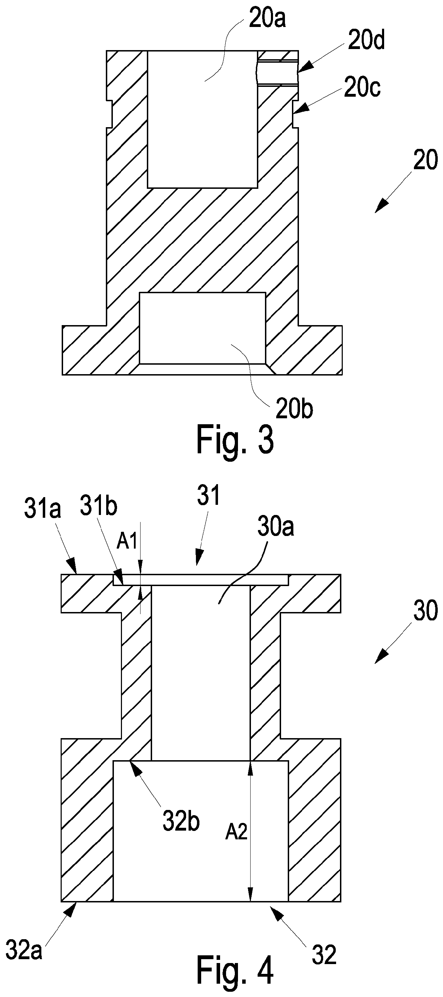

Die Figuren 3, 4 und 5 zeigen jeweils eine schematische Schnittdarstellung des Spritzendrückelements, der Halterung und des Führungselements gemäß der bevorzugten Ausführungsform der vorliegenden Erfindung.

Die Sensorvorrichtung 80 kann beispielsweise als Messgabel 81 mit Wägebrücke als Kraftaufnehmer ausgebildet sein, auf der die Halterung 30 oder eine spezielle Halterung aufliegt bzw. eingehängt ist. Beispielsweise kann die spezielle Halterung für eine Filterintegritätsprüfung verwendet werden, die im Gebrauch auf der Messgabel 81 aufliegen, wodurch während der Prüfung Kraftwerte aufgenommen werden können. Über eine entsprechende Elektronik können diese Werte in Druckwerte umgerechnet werden, wobei die Druckwerte während der Filterintegritätsprüfung dokumentiert werden können.

Die Halterung 30 kann über ein anderes Halteelement gehaltert sein, so dass bei einem Auspressvorgang während einer Filtration die Sensorvorrichtung 80 nicht verwendet wird.

Wie aus Figur 3 ersichtlich ist, weist Spritzendrückelement 20 die erste Vertiefung 20a und eine zweite Vertiefung 20b auf. Das Spritzendrückelement 20 weist ferner eine durch eine Umlaufnut gebildete seitliche farbige Markierung 20c und eine seitliche Bohrung 20d, in die die Gewindeschraube 21 geschraubt ist, auf. Diese Elemente wirken mit einer Vorschubvorrichtung 10 zusammen. Die durch eine Umlaufnut gebildete farbige Markierung 20c am Spritzendrückelement 20 ist in einem Zustand, in dem das Spritzendrückelement 20 in die Kraftspeichereinheit 14 eingeschoben ist, durch seitliche Bohrungen 12d am Führungselement 12 hindurch sichtbar. Dadurch kann festgestellt werden, ob das Kraftspeicherelement 13 ausreichend beladen ist und ein Ausgangszustand für einen Auspressvorgang erreicht ist. Durch die Markierung 20c kann auch der maximale Federweg des zusammengedrückten Kraftspeicherelement 13 von außen festgestellt werden und markiert somit das Ende des Federwegs des Kraftspeicherelements 13. Das Spritzendrückelement 20 stößt nun mit dem oberen Ende im Führungselement 12 an. Dieser Zustand bedeutet, dass mit dem Vorschub des Vorschubelements 11 gestoppt werden muss, da bei weiterem Vorschub die kraftaufnehmende Funktion des Kraftspeicherelements 13 durch Anstoßen des oberen Endes des Spritzendrückelements 20 in dem Führungselement 12 aufgehoben wird. In diesem Zustand wird der Vorschub des Vorschubelements 11 direkt auf die Spritze übertragen, wodurch zu hohe Kräfte auf den Spritzenkolben 42 wirken und somit zu hohe Druckverhältnisse in der gesamten Spritze und in einem eventuell vorgeschraubten Spritzenvorsatzfilter entstehen können, was sehr schnell zum Bersten einer dieser Komponenten führen würde.

In der zweiten Vertiefung 20b ist der Spritzenkolben 42 teilweise aufnehmbar, um ein Abrutschen während des Auspressvorgangs und eine Schräglage des Spritzenkolbens 42 in Bezug auf den Spritzenzylinder 41 zu vermeiden.

Wie aus Figur 4 ersichtlich ist, ist die Halterung 30 als zylindrisches Bauteil mit einer Durchgangsbohrung 30a gebildet und weist eine erste Seite 31 und eine zweite Seite 32 auf. Die erste Seite 31 weist einen ersten Anschlag 31a und eine erste Auflagefläche 31b auf, wobei zwischen dem ersten Anschlag 31a und der ersten Auflagefläche 31b ein erster Abstand A1 gebildet ist. Die zweite Seite 32 weist einen zweiten Anschlag 32a und eine zweite Auflagefläche 32b auf, wobei zwischen dem ersten Anschlag 32a und der zweiten Auflagefläche 32b ein zweiter Abstand A2 gebildet ist. Die Spritze 40 wird -je nach Ausrichtung der Halterung 30 - von einer der beiden Seiten der Halterung 30 in die Durchgangsbohrung 30a gesteckt, wobei der Durchmesser der Durchgangsbohrung 30a an den Durchmesser eines Kragens 43 am hinteren Ende des Spritzenzylinders 41 angepasst ist, sodass der Spritzenzylinder mit dem Kragen 43 auf der ersten oder zweiten Auflagefläche 31b bzw. 32b aufliegt und axial abgestützt ist. Während eines Auspressvorgangs kann der Spritzenkolben 42 soweit vom Spritzendrückelement 20 hinunter gedrückt werden, bis das Spritzendrückelement 20 mit dem jeweiligen ersten bzw. zweiten Anschlag 31a, 32a in Kontakt kommt.

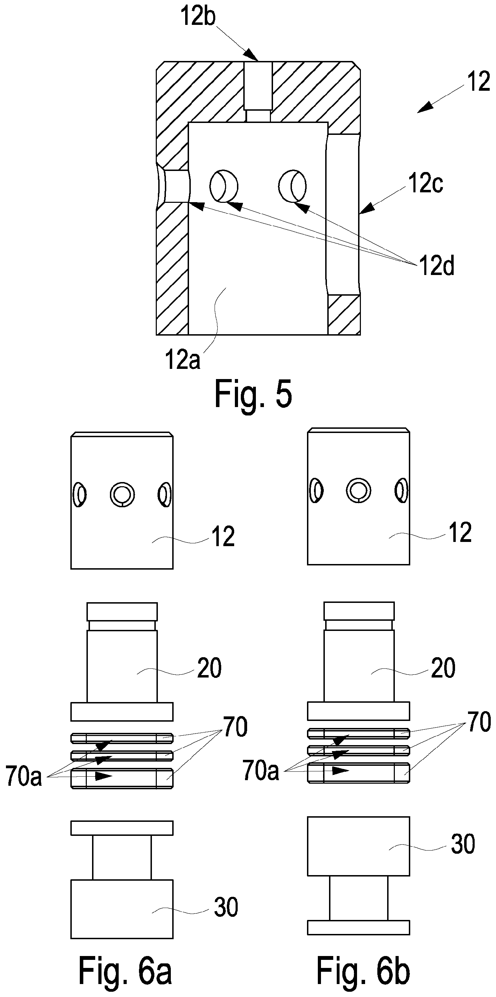

Wie in Figur 5 gezeigt ist, weist das Führungselement 12 einen Aufnahmeraum 12a auf, in dem das Kraftspeicherelement 13 angeordnet ist, und in dem in Gebrauch das Spritzendrückelement 20 teilweise aufgenommen und geführt ist. Das Führungselement 12 weist ferner eine axiale Bohrung 12b zur Befestigung des Vorschubelements 11, das seitlich positioniertes und sich in axialer Richtung erstreckende Langloch 12c und die mehrere seitliche Bohrungen 12d auf.

Figur 6a und 6b zeigen die möglichen Ausrichtungen der Halterung 30 und mögliche Anordnungen von Distanzscheiben 70 nach dem bevorzugten Ausführungsbeispiel der vorliegenden Erfindung.

Wie in den Figuren 6a und 6b dargestellt, kann die Halterung 30 bezogen auf deren axiale Richtung derart ausgerichtet sein, dass die Spritze 40 mit dem Kragen 43 an der ersten Auflagefläche 31b anliegt (Ausrichtungsvariante A, vgl. Fig. 6a) oder derart ausgerichtet sein, dass die Spritze 40 an der zweiten Auflagefläche 32b anliegt (Ausrichtungsvariante B, vgl. Fig. 6b). Ferner sind, wie aus den Figuren 6a und 6b ersichtlich, Distanzscheiben 70 mit definierten Dicken zwischen dem Spritzendrückelement 20 und der Halterung 30 anordenbar, wobei eine oder mehrere Distanzscheiben 70 auf die erst oder zweite Auflagefläche 31b, 32b aufgelegt werden. Durch das Vorsehen einer geeigneten Distanzscheibe 70, oder einer geeigneten Kombination von Distanzscheiben 70, kann der Arbeitsbereich der Auspressvorrichtung 100 eingestellt werden, d.h. es kann bestimmt werden wie weit eine Spritze 40 ausgepresst wird. Insbesondere kann dadurch vermieden werden, dass der Spritzenkolben 42 der Spritze 40 weiter hinunter gedrückt wird, als gewünscht. Mit anderen Worten: Durch Anordnen der Halterung 30 in einer gewünschten Ausrichtungsvariante A oder B sowie durch das Vorsehen von Distanzscheiben 70 kann bei vorgegebener Füllmenge einer Spritze 40 die auszupressende Menge eines in der Spritze 40 befindlichen Fluides festgelegt werden.

Beispielsweise kann abhängig von der Wahl und Anordnung der Distanzscheiben 70 und der Ausrichtung der Halterung 30 eine Menge von 1, 2, 3, 4, 5, 6, 7, 8, 9 oder 10 Millilitern eines in der Spritze 40 befindlichen Fluides bei vorgegebener Füllmenge von 10 Millilitern ausgepresst werden.

Vorzugsweise können bei der Ausrichtungsvariante A der Halterung 30 ohne Distanzscheibe 10 Milliliter, mit einer oder mehreren Distanzscheiben 70 mit einer (Gesamt-)dicke von 5 Millimetern 9 Milliliter, mit einer oder mehreren Distanzscheiben 70 mit einer (Gesamt-)dicke von 10 Millimetern 8 Milliliter, mit einer oder mehreren Distanzscheiben 70 mit einer (Gesamt-)dicke von 15 Millimetern 7 Milliliter, und mit einer oder mehreren Distanzscheiben 70 mit einer (Gesamt-)dicke von 20 Millimetern 6 Milliliter eines in der Spritze 40 befindlichen Fluides ausgepresst werden.

Vorzugsweise können bei der Ausrichtungsvariante B der Halterung 30 ohne Distanzscheibe 5 Milliliter, mit einer oder mehreren Distanzscheiben 70 mit einer (Gesamt-)dicke von 5 Millimetern 4 Milliliter, mit einer oder mehreren Distanzscheiben 70 mit einer (Gesamt-)dicke von 10 Millimetern 3 Milliliter, mit einer oder mehreren Distanzscheiben 70 mit einer (Gesamt-)dicke von 15 Millimetern 2 Milliliter, und mit einer oder mehreren Distanzscheiben 70 mit einer (Gesamt-)dicke von 20 Millimetern 1 Milliliter eines in der Spritze 40 befindlichen Fluides ausgepresst werden.

Somit können mit drei Distanzscheiben 70, wobei eine Distanzscheibe 70 eine Dicke von 10 Millimetern und zwei Distanzscheiben 70 eine Dicke von 5 Millimetern aufweisen, alle Kombinationen von 1 bis 10 Millilitern eines Fluides aus der Spritze 40 gedrückt werden.

### Bezugszeichenliste:

10 Vorschubvorrichtung
11 Vorschubelement
12 Führungselement
12a Aufnahmeraum
12b Bohrung
12c Langloch
12d Bohrung
13 Kraftspeicherelement
14 Kraftspeichereinheit
20 Spitzendrückelement
20a Vertiefung
20b Vertiefung
20c Markierung
20d Bohrung
21 Gewindeschraube
30 Halterung
30a Durchgangsbohrung
31 erste Seite
31a erster Anschlag
31b erste Auflagefläche
32 zweite Seite
32a zweiter Anschlag
32b zweite Auflagefläche
40 Spritze
41 Spritzenzylinder
42 Spritzenkolben
43 Kragen
50 Betätigungsvorrichtung
60 Halterungsvorrichtung
61 Ständerung
62 Standfuß
63 Träger
64 Klemmschraube
66 Nut
68 Anschlageinrichtung
70 Distanzscheibe
70a Aussparung
80 Sensorvorrichtung
81 Messgabel
100 Auspressvorrichtung
A1 Abstand
A2 Abstand

## Patentansprüche

1. Auspressvorrichtung (100) für Spritzen (40) mit einem Spritzenzylinder (41) und einem Spritzenkolben (42),
mit einer Halterung (30) zum Haltern einer Spritze (40), wobei die Halterung (30) den Spritzenzylinder (41) der Spritze (40) zumindest teilweise umgreift,
mit einem Spritzendrückelement (20), das am freien Ende des Spritzenkolbens (42) angreift, wobei der Spritzenkolben (42) zum Auspressen der Spritze (40) durch das Spritzendrückelement (20) drückbar ist,
mit einer Vorschubvorrichtung (10) zur Aufbringung einer kontinuierlich auf das Spritzendrückelement (20) wirkenden Auspresskraft zum Auspressen der Spritze (40), wobei die Vorschubvorrichtung (10) das Spritzendrückelement (20) vorschiebt, und mit einer Betätigungsvorrichtung (50) zur Betätigung der Vorschubvorrichtung (10),
**dadurch gekennzeichnet,**
**dass** die Vorschubvorrichtung (10) ein Vorschubelement (11) und eine Kraftspeichereinheit (14) mit einem Kraftspeicherelement (13) aufweist, wobei das Vorschubelement (11) die Kraftspeichereinheit (14) betätigt, um eine Kraft in das Kraftspeicherelement (13) einzuleiten, wobei das Kraftspeicherelement (13) die Kraft anschließend kontinuierlich zur Erzeugung der Auspresskraft zum Auspressen der Spritze (40) freigibt.

2. Auspressvorrichtung nach Anspruch 1, **dadurch gekennzeichnet, dass** die Halterung (30) eine erste Auflagefläche (31b) aufweist, an der die Spritze (40) mit einem vorderen Ende des Spritzenzylinders (41) oder mit einem Kragen (43) am hinteren Ende des Spritzenzylinders (41) aufliegen kann, wobei die Halterung (30) eine Durchgangsbohrung (30a) zur Aufnahme der Spritze (40) aufweist, wobei die erste Auflagefläche (31b) in der Durchgangsbohrung (30a) angeordnet ist.

3. Auspressvorrichtung nach Anspruch 2, **dadurch gekennzeichnet, dass** die Halterung (30) einen ersten Anschlag (31a) aufweist, wobei zwischen dem ersten Anschlag (31a) und der ersten Auflagefläche (31b) in einer ersten Richtung ein erster Abstand (A1) gebildet ist.

4. Auspressvorrichtung nach Anspruch 2 oder 3, **dadurch gekennzeichnet, dass** die Halterung (30) eine zweite Auflagefläche (32b) aufweist, an der die Spritze (40) mit einem vorderen Ende des Spritzenzylinders (41) oder mit einem Kragen (43) am hinteren Ende des Spritzenzylinders (41) aufliegen kann, wobei die zweite Auflagefläche (32b) in der Durchgangsbohrung (30a) angeordnet ist.

5. Auspressvorrichtung nach Anspruch 4, **dadurch gekennzeichnet, dass** die Halterung (30) einen zweiten Anschlag (32a) aufweist, wobei zwischen dem zweiten Anschlag (32a) und der zweiten Auflagefläche (32b) in der ersten Richtung ein zweiter Abstand (A2) gebildet ist, wobei der zweite Abstand (A2) größer als der erste Abstand (A1) ist.

6. Auspressvorrichtung nach einem der Ansprüche 1 bis 5, **dadurch gekennzeichnet, dass** zwischen der Halterung (30) und dem Spritzendrückelement (20) eine oder mehrere Distanzscheiben (70) mit einer jeweils definierten Dicke und mit einer Aussparung (70a) anordenbar sind, wobei die eine oder mehreren Distanzscheiben (70) an der Halterung (30) anordenbar sind und die eine oder mehreren Distanzscheiben (70) auf dem ersten oder dem zweiten Anschlag (31a, 32a) auflegbar sind.

7. Auspressvorrichtung nach einem der Ansprüche 1 bis 6, **dadurch gekennzeichnet, dass** das Vorschubelement (11) durch eine Vorschubstange gebildet ist.

8. Auspressvorrichtung nach Anspruch 6 oder 7, **dadurch gekennzeichnet, dass** die Kraftspeichereinheit (14) ein Führungselement (12) für das Spritzendrückelement (20) aufweist und/oder das Spritzendrückelement (20) eine erste Vertiefung (20a) aufweist, in der das Kraftspeicherelement (13) zumindest teilweise aufgenommen ist.

9. Auspressvorrichtung nach Anspruch 8, **dadurch gekennzeichnet, dass** das Führungselement (12) einen Aufnahmeraum (12a) aufweist, in dem das Spritzendrückelement (20) teilweise aufgenommen und geführt ist, wobei das Führungselement (12) ein sich in axialer Richtung erstreckendes seitlich angeordnetes Langloch (12c) aufweist, das in den Aufnahmeraum (12a) mündet, und das Spritzendrückelement (20) eine seitliche Bohrung (20d) aufweist, wobei das Spritzendrückelement (20) durch ein in der seitlichen Bohrung (20d) angeordnetes Führungsteil im Langloch (12c) geführt ist.

10. Auspressvorrichtung nach einem der Ansprüche 1 bis 9, **dadurch gekennzeichnet, dass** das Spritzendrückelement (20) eine zweite Vertiefung (20b) aufweist, in der der Spritzenkolben (42) teilweise aufgenommen ist.

11. Auspressvorrichtung nach einem der Ansprüche 1 bis 10, **gekennzeichnet durch** eine Sensorvorrichtung (80) zur Bestimmung der Auspresskraft (F), wobei die Sensorvorrichtung (80) an einer Halterungsvorrichtung (60) oder an der Halterung (30) oder an dem Spritzendrückelement (20) angeordnet ist.

12. Auspressvorrichtung nach einem der Ansprüche 1 bis 11, **dadurch gekennzeichnet, dass** das Spritzendrückelement (20) eine Markierung (20c) zur Überwachung eines Auspressvorgangs und/oder zur Anzeige eines Anfangszustandes für einen Auspressvorgang aufweist, wobei die von außen sichtbare Markierung (20c) durch eine Umlaufnut gebildet ist.

13. Auspressvorrichtung nach Anspruch 12, **dadurch gekennzeichnet, dass** das Führungselement (12) mindestens eine seitliche in den Aufnahmeraum (12a) mündende Bohrung (12d) aufweist, die mit der Markierung (20c) zusammenwirkt, wobei die relative Position der Markierung (20c) bzw. des Spritzendrückelements (20) in Bezug auf das Führungselement (12) durch die seitlichen Bohrungen (12d) sichtbar ist.

14. Auspressvorrichtung nach einem der Ansprüche 1 bis 13, **dadurch gekennzeichnet, dass** die Betätigungsvorrichtung (50) eine elektrische Antriebseinheit aufweist, die die Vorschubvorrichtung (10) betätigt, wobei die Vorschubvorrichtung (10) eine Gewindespindel aufweist, die von der elektrischen Antriebseinheit angetrieben wird und das Spritzendrückelement (20) vorschiebt.

15. Verwendung einer Auspressvorrichtung nach einem der Ansprüche 1 bis 14 zur Durchführung einer Sterilfiltration eines in einer Spritze (40) befindlichen Fluides und/oder zur Durchführung eines Bubble-Point Tests.

## Claims

1. Dispensing device (100) for syringes (40) comprising a syringe cylinder (41) and a syringe plunger (42),
comprising a holder (30) for holding a syringe (40), wherein the holder (30) at least partially surrounds the syringe barrel (41) of the syringe (40),
comprising a syringe plunger (20) which engages the free end of the syringe plunger (42), wherein the syringe plunger (42) can be pushed by the syringe plunger (20) to expel the contents of the syringe (40),
with a feed device (10) for applying a continuous extrusion force acting on the syringe plunger element (20) to extrude the syringe (40), wherein the feed device (10) advances the syringe plunger element (20), and with an actuating device (50) for actuating the feed device (10),
**characterised in**
**that** the feed device (10) comprises a feed element (11) and a force storage unit (14) with a force storage element (13), wherein the feed element (11) actuates the energy storage unit (14) to impart a force to the energy storage element (13), wherein the energy storage element (13) subsequently releases the force continuously to generate the ejection force for ejecting the syringe (40).

2. A dispensing device according to claim 1, **characterised in that** the holder (30) comprises a first bearing surface (31b) against which the syringe (40) can rest against with a front end of the syringe cylinder (41) or with a collar (43) at the rear end of the syringe cylinder (41), wherein the holder (30) comprises a through-bore (30a) for receiving the syringe (40), wherein the first support surface (31b) being arranged in the through-hole (30a).

3. Extrusion device according to claim 2, **characterised in that** the holder (30) has a first stop (31a), wherein a first distance (A1) is formed between the first stop (31a) and the first bearing surface (31b) in a first direction.

4. Extrusion device according to claim 2 or 3, **characterised in that** the holder (30) comprises a second bearing surface (32b) against which the syringe (40) can rest with a front end of the syringe barrel (41) or with a collar (43) at the rear end of the syringe barrel (41), wherein the second bearing surface (32b) is arranged in the through-bore (30a).

5. Ejection device according to claim 4, **characterised in that** the holder (30) comprises a second stop (32a), wherein a second distance (A2) is formed between the second stop (32a) and the second bearing surface (32b) in the first direction, wherein the second distance (A2) is greater than the first distance (A1).

6. Extrusion device according to any one of claims 1 to 5, **characterised in that** one or more spacer discs (70), each having a defined thickness and a recess (70a), wherein the one or more spacer discs (70) can be arranged on the holder (30) and the one or more spacer discs (70) can be placed on the first or second stop (31a, 32a).

7. Ejection device according to any one of claims 1 to 6, **characterised in that** the feed element (11) is formed by a feed rod.

8. An extrusion device according to claim 6 or 7, **characterised in that** the force storage unit (14) comprises a guide element (12) for the nozzle push-back element (20) and/or the nozzle push-back element (20) comprises a first recess (20a) in which the force storage element (13) is at least partially received.

9. Ejection device according to claim 8, **characterised in that** the guide element (12) comprises a receiving chamber (12a) in which the nozzle-pushing element (20) is partially received and guided, wherein the guide element (12) comprises a laterally arranged elongated hole (12c) which opens into the receiving space (12a), and the syringe plunger element (20) comprises a lateral bore (20d), wherein the syringe plunger element (20) is guided in the elongated hole (12c) by a guide part arranged in the lateral bore (20d).

10. Extrusion device according to any one of claims 1 to 9, **characterised in that** the syringe plunger element (20) comprises a second recess (20b) in which the syringe plunger (42) is partially received.

11. Extrusion device according to any one of claims 1 to 10, **characterised by** a sensor device (80) for determining the extrusion force (F), wherein the sensor device (80) is arranged on a mounting device (60) or on the holder (30) or on the syringe plunger element (20).

12. Extrusion device according to any one of claims 1 to 11, **characterised in that** the syringe plunger element (20) comprises a marking (20c) for monitoring an extrusion process and/or for indicating an initial state for an extrusion process, wherein the marking (20c), which is visible from the outside, is formed by a circumferential groove.

13. Extrusion device according to claim 12, **characterised in that** the guide element (12) comprises at least one lateral bore (12d) opening into the receiving chamber (12a), which cooperates with the marking (20c), wherein the relative position of the marking (20c)
or of the spray-trigger element (20) relative to the guide element (12) is visible through the lateral bores (12d).

14. Extrusion device according to any one of claims 1 to 13, **characterised in that** the actuating device (50) comprises an electric drive unit which actuates the feed device (10), wherein the feed device (10) comprises a threaded spindle which is driven by the electric drive unit and feeds the syringe plunger element (20).

15. Use of a syringe-emptying device according to any one of claims 1 to 14 for performing sterile filtration of a fluid contained in a syringe (40) and/or for performing a Bubbie-Point test.

## Revendications

1. Dispositif de compression (100) pour seringues (40) comprenant un cylindre de seringue (41) et d'un piston de seringue (42),
comprenant un support (30) destiné à maintenir une seringue (40), le support (30) entourant au moins partiellement le cylindre (41) de la seringue (40),
comprenant un élément de poussée de seringue (20) qui vient en prise avec l'extrémité libre du piston de seringue (42), le piston de seringue (42) pouvant être poussé par l'élément de poussée de seringue (20) pour vider la seringue (40),
avec un dispositif d'avance (10) destiné à exercer une force d'expulsion agissant en continu sur l'élément de poussée de la seringue (20) pour vider la seringue (40), le dispositif d'avance (10) faisant avancer l'élément de poussée de la seringue (20), et avec un dispositif d'actionnement (50) destiné à actionner le dispositif d'avance (10),
**caractérisé en ce**
**que** le dispositif d'avance (10) comprend un élément d'avance (11) et une unité d'accumulation de force (14) avec un élément d'accumulation de force (13), l'élément d'avance (11) actionne l'unité d'accumulation d'énergie (14) afin d'introduire une force dans l'élément d'accumulation d'énergie (13), l'élément d'accumulation d'énergie (13) libérant ensuite cette force de manière continue pour générer la force d'éjection permettant d'éjecter la seringue (40).

2. Dispositif d'extrusion selon la revendication 1, **caractérisé en ce que** le support (30) comporte une première surface d'appui (31b) sur laquelle la seringue (40) peut reposer avec une extrémité avant du cylindre de la seringue (41) ou avec un collet (43) à l'extrémité arrière du cylindre de la seringue (41), le support (30) comportant un alésage traversant (30a) destiné à recevoir la seringue (40), la première surface d'appui (31b) étant disposée dans le trou traversant (30a).

3. Dispositif d'extraction selon la revendication 2, **caractérisé en ce que** le support (30) comporte une première butée (31a), une première distance (A1) étant formée entre la première butée (31a) et la première surface d'appui (31b) dans une première direction.

4. Dispositif d'extrusion selon la revendication 2 ou 3, **caractérisé en ce que** le support (30) comporte une deuxième surface d'appui (32b) sur laquelle la seringue (40) peut reposer par une extrémité avant du cylindre de la seringue (41) ou par un collet (43) à l'extrémité arrière du cylindre de la seringue (41), la deuxième surface d'appui (32b) étant disposée dans l'alésage traversant (30a).

5. Dispositif d'extrusion selon la revendication 4, **caractérisé en ce que** le support (30) comporte une deuxième butée (32a), une deuxième distance (A2) étant formée entre la deuxième butée (32a) et la deuxième surface d'appui (32b) dans la première direction, la deuxième distance (A2) étant supérieure à la première distance (A1).

6. Dispositif d'extrusion selon l'une des revendications 1 à 5, **caractérisé en ce qu'**une ou plusieurs rondelles d'écartement (70) présentant chacune une épaisseur définie et un évidement (70a), la ou les rondelles d'écartement (70) pouvant être disposées sur le support (30) et pouvant être placées sur la première ou la deuxième butée (31a, 32a).

7. Dispositif d'extrusion selon l'une des revendications 1 à 6, **caractérisé en ce que** l'élément d'avance (11) est constitué d'une tige d'avance.

8. Dispositif d'extraction selon la revendication 6 ou 7, **caractérisé en ce que** l'unité d'accumulation de force (14) comporte un élément de guidage (12) pour l'élément de poussée d'extrémité (20) et/ou l'élément de poussée d'extrémité (20) comporte un premier renfoncement (20a) dans lequel l'élément d'accumulation d'énergie (13) est au moins partiellement logé.

9. Dispositif d'éjection selon la revendication 8, **caractérisé en ce que** l'élément de guidage (12) comporte un espace de logement (12a) dans lequel l'élément de poussée de l'éjecteur (20) est partiellement logé et guidé, l'élément de guidage (12) comportant un trou oblong s'étendant dans la direction axiale et disposé latéralement (12c) s'étendant dans la direction axiale et disposé latéralement, qui débouche dans l'espace de logement (12a), et l'élément de poussée de la seringue (20) comporte un alésage latéral (20d), l'élément de poussée de la seringue (20) étant guidé dans l'alésage oblong (12c) par une pièce de guidage disposée dans l'alésage latéral (20d).

10. Dispositif d'extrusion selon l'une des revendications 1 à 9, **caractérisé en ce que** l'élément de poussée de la seringue (20) comporte un deuxième renfoncement (20b) dans lequel le piston de la seringue (42) est partiellement logé.

11. Dispositif d'extrusion selon l'une des revendications 1 à 10, **caractérisé par** un dispositif capteur (80) destiné à déterminer la force d'extrusion (F), le dispositif capteur (80) étant disposé sur un dispositif de fixation (60) ou sur le support (30) ou sur l'élément de poussée de la seringue (20).

12. Dispositif d'extrusion selon l'une des revendications 1 à 11, **caractérisé en ce que** l'élément de poussée de la seringue (20) comporte un repère (20c) destiné à surveiller un processus d'extrusion et/ou à indiquer un état initial pour un processus d'extrusion, le repère (20c) visible de l'extérieur étant formé par une rainure périphérique.

13. Dispositif d'extrusion selon la revendication 12, **caractérisé en ce que** l'élément de guidage (12) comporte au moins un alésage latéral (12d) débouchant dans l'espace de réception (12a), qui coopère avec le repère (20c), la position relative du repère (20c) ou de l'élément de poussée de l'injecteur (20) par rapport à l'élément de guidage (12) est visible à travers les alésages latéraux (12d).

14. Dispositif d'extrusion selon l'une des revendications 1 à 13, **caractérisé en ce que** le dispositif d'actionnement (50) comprend une unité d'entraînement électrique qui actionne le dispositif d'avance (10), le dispositif d'avance (10) comprenant une vis filetée qui est entraînée par l'unité d'entraînement électrique et qui fait avancer l'élément de poussée de la seringue (20).

15. Utilisation d'un dispositif d'extrusion selon l'une des revendications 1 à **14** pour effectuer une filtration stérile d'un fluide se trouvant dans une seringue (40) et/ou pour effectuer un test de point de bulle.
